# EUROPEAN PATENT SPECIFICATION

(11) **EP 0 801 082 B1**
(45) Date of publication and mention of the grant of the patent: **09.04.2003**
(21) Application number: 97111264.4
(22) Date of filing: 27.08.1991
(51) Int. Cl.: C08F 8/00, C07K 1/04

(54) **Method for making graft resins for solid-phase peptide synthesis**
Verfahren zur Herstellung von Harzen für die Feststoff-Peptidsynthese
Procédé pour la préparation des résines pour la synthèse de peptides en phase solide

(30) Priority: 31.08.1990 US 576314; 14.06.1991 US 715289
(43) Date of publication of application: 15.10.1997
(62) Divisional of application: 95112933.7
(73) Proprietor: REGENTS OF THE UNIVERSITY OF MINNESOTA, Minneapolis, Minnesota 55415-1226 (US)
(72) Inventor: Barany, George, Falcon Heights, MN 55113 (US); Chang, Jane, Buffalo Grove, IL 60089 (US); Sole, Nuria A., Medford, MA 02155 (US); Albericio, Fernando, 142 E-08028 Barcelona (ES); Zalipski, Samuel, Menlo Park, California 94025 (US)
(74) Representative: Kirkham, Nicholas Andrew

(56) References cited:
- EP-A- 0 187 391
- EP-A- 0 347 145
- CH-A- 602 822
- DE-A- 2 649 848
- US-A- 4 831 084

## Description

Spacer arms are essential in many areas of modern biochemistry. Spacer arms can be defined as molecules which link one molecule to another molecule or to an inert support. Polyethylene glycol, for example, has been used advantageously to link enzymes to insoluble carriers and other biomolecules while retaining the activity of the enzyme. M. Stark and K. Holmberg, Biotech. and Bioeng., 34:942-950 (1989). This concept has important consequences for industrial processes using immobilized enzymes (e.g., affinity column purification processes), and for diagnostic assays (e.g., ELISA (Enzyme linked immuno sorbant assay) assays). Two other areas in which polyethylene glycol spacer arms have been used are peptide synthesis and sequencing. The coupling rate of protected nucleotide and amino acid residues to inert supports, such as silica, membrane, and polystyrene supports, often increases commensurately with the separation of the reaction site from the support backbone. Similar effects have been shown for sequencing of solid-phase immobilized samples. J.K. Inman et al., In Solid Phase Methods in Protein Sequence Analysis, Previero and Coletti-Previero, (Eds.), Elsevier, North-Holland Biomed. Press, pp. 81-94 (1977).

The effectiveness of solid-phase nucleic acid or peptide synthesis or sequence analysis is affected by the solid-phase or support which anchors the reactive sites. Polystyrene gels or porous glass have both been utilized as solid supports for peptide sequencing, for example. In many applications, the solvents used in the process can cause the polystyrene particles to change in volume, which causes blocking of the reaction column and back pressure. Conversely, porous glass is completely rigid and does not change in volume, but the chemical properties of porous glass derivatives have lacked reproducibility. Polymer particles, such as polystyrene particles, which have been derivatized so that reactive groups can be attached to them, have proved useful in many applications. Polyethylene glycol (PEG) structures have been used as chemically inert spacer arms because they are compatible with a wide range of solvents. Inman et al., ibid. The use of PEG spacer arms minimizes the steric effects caused by the support. PEG spacer arms provide another useful function in modifying the character of the pore space so that the support-bound reactive moiety is compatible with a wider range of solvents and reagents.

PEG-modified polystyrene (PEG-PS) resins have been described for use in solid-phase peptide sequencing. Inman et al., ibid. PEG-PS resins have also been utilized as phase transfer catalysts. W.M. McKenzie et al., J. Chem. Soc. Chem. Commun., p. 541-543 (1978); S.L. Regen et al., J. Amer. Chem. Soc., 101:116-120 (1979); J.G. Heffernan et al., J. Chem. Soc. Perkin II, p. 514-517 (1981); Y. Kimura et al., J. Org. Chem., 48:385-386 (1983); H. Tomoi et al., Reactive Polymers, 10:27-36 (1989). PEG-PS resins have been described as supports for solid-phase peptide synthesis. Becker et al., Makromol. Chem. Rapid Commun., 3:217-223 (1982); H. Hellermann, et al., Makromol. Chem., 184:2603-2617 (1983). However, PEG-PS resins prepared by the referenced methods suffer from several drawbacks. The reactions proceeded poorly with high molecular weight PEG (e.g., greater than 400 daltons) and symmetrical bifunctional PEG tended to form crosslinks. These problems were reduced by the anionic polymerization of ethylene oxide directly onto crosslinked polystyrene. Bayer et al., In Peptides: Structure and Function, V.J. Hruby and D.H. Rich (eds.), Proc. 8th Am. Peptide Symp. pp. 87-90, Pierce Chem. Co., Rockford, IL (1983). Bayer and Rapp, German Patent DE 3500180 A1 (1986). However, the PEG chain lengths are difficult to control using this method, and the uniformity of the PEG polymers is uncertain. Another problem with this process is that the polystyrene is functionalized using chloromethyl ether, which is highly toxic, and residual chloromethyl groups can cause side reactions during peptide synthesis.

Another method of making PEG graft copolymers is described by Zalipsky et al., In Peptides:Structure and Function, G.M. Deber, V.J. Hruby and K.D. Kopple (eds.), Proc. 9th Am. Pep. Symp., pp. 257-260, Pierce Chem. Co., Rockford, IL (1985). In this method, certain heterobifunctional PEG derivatives of defined molecular weight (i.e., 2000 to 4000 daltons) were used. However, these derivatives are not readily available, which hinders their commercialization.

A method of preparing non-toxic and efficient solid supports which can be used with a wide range of solvents would be valuable for use in solid-phase synthesis or sequencing of peptides or nucleic acids, or for other solid-phase applications.

### Summary of the Invention

The present invention pertains to a method for making polyethylene glycol derivatized graft supports. The specification discloses polyethylene glycol derivatized graft supports and methods of using the supports to synthesize peptides by solid-phase synthesis techniques. The PEG-graft supports prepared by the method of this invention comprise functionalized PEG derivatives which are covalently attached to solid supports.

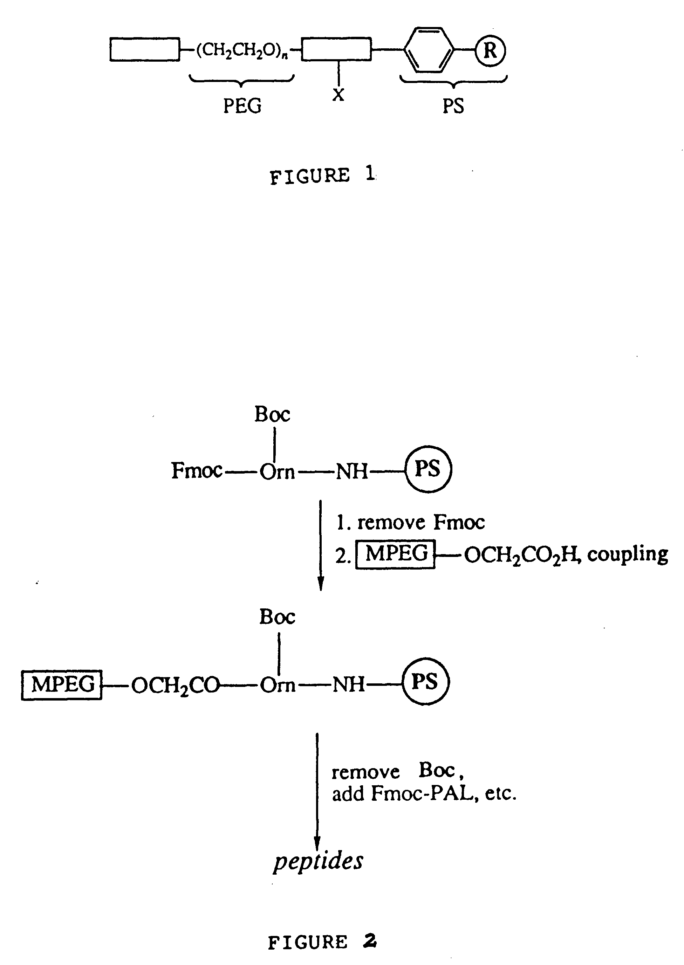

This invention provides a method of making a resin that permits peptide synthesis to occur at point other than the terminus of PEC. Such resins are illustrated in Figure 1 and further represented by the general Formula II: 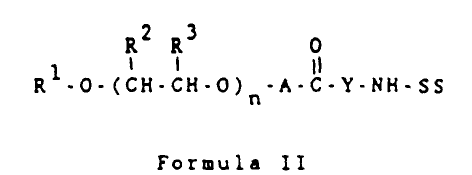 wherein Y is a diamino monocarboxylic acid residue that can optionally be protected by a N^{ω} protecting group; n is an integer from 5 to 150; SS is a solid support; A is a straight chain or branched C1-C10 alkyl group, such as methylene, ethylene, propylene, isopropylene, butylene and isobutylene; and R¹, R² and R³ are independently selected from the group consisting of hydrogen, alkyl groups and aryl groups.

The resin of Formula II, as defined in the preceding paragraph is the subject of the claims of our EP-B-0 687 691.

The present invention provides a compact, commercially viable route for making functionalized inert supports for solid-phase applications using monofunctional or homobifunctional polyethylene glycol as the starting material. The present PEG graft supports provide advantageous physical and mechanical properties for solid-phase peptide synthesis, nucleic acid synthesis, and other applications where immobilized molecules are used.

### Brief Description of the Figures

Figure 1 shows the general structure of a polyethylene glycol-polystyrene (PEG-PS) graft support. X illustrates the point from which biopolymer chain growth begins.

Figure 2 is a schematic shoving a series of reactions leading to a PEG-PS graft support in which the biopolymer can be synthesized from the Orn residue.

### Detailed Description of the Invention

The present invention pertains to methods of making resins comprising a functionalized polyethylene glycol derivatives covalently linked to a solid support. As disclosed and claimed in patent EP-B-0 546 055, one such graft support comprises a symmetrical poly(oxyethylene) diamine derivative linked to the support and is represented by Formula I: 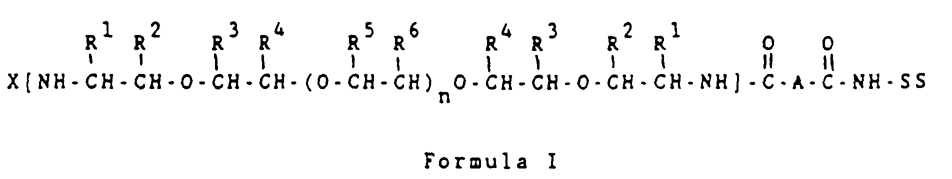 wherein n is an integer from 5 to about 150; R¹, R², R³, R⁴, R⁵ and R⁶ are independently selected from the group consisting of hydrogen (H), and simple alkyl or aryl groups such as methyl, ethyl or phenyl; SS is a solid support; X is H or H₂N-B-NH-C(O)-A-C(O)-; and A and B are independently a straight chain or branched alkyl group, such as ethylene, propylene, isopropylene, butylene, isobutylene or the like up to C-10 in length (e.g., A derived from succinic, glutaric, adipic or other such acids; B derived from ethylenediamine or other aliphatic diamines); a CH-CH group (e.g., A derived from maleic acid) or an aromatic group (e.g., A derived from phthalic acid; B derived from phenylenediamine). The core portion of the formula, within the bracketed portion, corresponds to a series of polyoxyethylene diamine polymers. The amino group(s) can optionally be protected by known N^{ω} protecting groups.

One method for producing graft supports of the invention is by reacting amino-functionalized core polymers with dicarboxylic acid derivatives, including anhydrides, to produce carboxyl-functional molecules. Dicarboxylic acids which are useful in this method include alkyl diacids having up to about 12 carbon atoms, such as, for example, maleic, succinic, glutaric or adipic acid; anhydrides such as maleic, succinic or glutaric anhydride; or aromatic anhydrides, such as phthalic anhydride. As disclosed in patent EP-B-0546055, the diamine polymer is reacted with succinic, maleic or glutaric anhydride to make representatives of the compounds claimed in patent EP-B-0546055, bis(succinylated), bis(maleylated) or bis(glutarylated) PEG. Figures 2 and 3 in EP-B-0 546 055 illustrate the formation and subsequent coupling of these derivatives onto an amino-functionalized solid support. For some applications, symmetrical dicarboxylic acid-functionalized polymers having approximately the same general structure can be used.

Carboxyl-functionalized spacer arm linkers produced by the present method are then coupled to appropriate parent carriers which have been functionalized with amino groups. Carriers which are useful as solid phases in the present invention include macromolecules or solids, such as membranes, porous glass, silica, polystyrenes, polydimethylacrylamides, cotton or paper. Solid supports which are particularly useful include amino-functionalized polystyrene, aminomethyl polystyrene, aminoacyl polystyrene and p-methylbenzhydrylamine polystyrene. A particularly preferred support is an amino-functionalized polystyrene-co-1% divinylbenzene. All amino groups of the parent carrier can be covered by reacting one equivalent of the carrier based on the amino groups with an excess of the carboxyl-functionalized derivatives. Most of the amino groups on the carrier become substituted by the polyethylene glycol derivatives, thereby forming spacer arms having one free pendant carboxyl group.

Introduction of an amino functionality on the present PEG derivatives is desirable for many synthesis applications. This can be achieved by acid hydrolysis (see Figure 2 of patent EP-B-0 546 055) of the amide moiety, thereby exposing the amino group which was originally present on the diamine, or by further coupling of a free or monoprotected low molecular-weight diamine (e.g., ethylene or hexamethylene diamine) with the carboxylate end group (see Figure 3 of patent EP-B-0 546 055). For parent carriers, modified by bis(maleylated) PEG linkers (see Figure 2 of parent EP-B-0 546 055), for example, the terminal pendant maleyl group is selectively hydrolyzed by controlled treatment with an acid, e.g., trifluoroacetic acid or dilute hydrochloric acid (HCl), whereas the other maleyl group, now linking the PEG to the carrier, is essentially stable. By these methods, amino-functionalized PEG-modified materials are obtained rapidly, efficiently and economically.

In the present method of making PEG-PS graft co-polymers, the diacid or anhydride is first reacted with the amino-functionalized support, thereby forming an amide-linked carboxyl functionalized support. This carboxyl functionalized support is activated and then contacted with an excess of a bifunctional diamine polymer, represented by the core structure described above.

The PEG graft supports of patent EP-B-0 546 055 makes it possible to assemble biopolymers at the PEG terminus (as shown in its Figure 1A). In the present invention, however, the resins can be constructed such that peptide synthesis can occur at a point other than the PEG terminus. Such resins are illustrated in Figure 1 hereof and are further represented by the general Formula II: 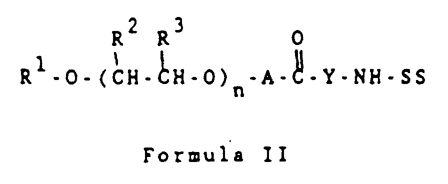 wherein Y is a diamino monocarboxylic acid residue that can optionally be protected by a N^{ω} protecting group; n is an integer from about 5 to about 150; SS is a solid support; A is a straight chain or branched C1-C10 alkyl group, such as methylene, ethylene, propylene, isopropylene, butylene and isobutylene; and R¹, R² and R³ are independently selected from the group consisting of hydrogen, alkyl groups and aryl groups.

A PEG graft support of this type (Formula II) is synthesized using a monofunctionalized polyethylene glycol (e.g., PEG monoamine or PEG monomethyl ether) which has been derivatized. Commercially available PEG starting materials are either monofunctional Jeffamine® or PEG monomethyl ether which has methoxy and hydroxyl end groups. Polymers which are particularly useful for this purpose include a series of poly(oxyethylene) diamines having a molecular weight up to about 6000 daltons which are commercially available under the tradename Jeffamine® (Texaco Chemical Co., Bellaire, TX). The Jeffamine® poly(oxyethylene) diamine resins are aliphatic primary diamines structurally derived from polypropylene oxide-capped polyethylene glycol.

An amino group on PEG can be reacted with a dicarboxylic acid or anhydride as already described. Alternatively, carboxyl-functionalized derivatives of MPEG can be made by reaction with ethyl bromoacetate. 4-bromovalerate or isocyanacetate, followed by saponification, for example as shown in the equation below.

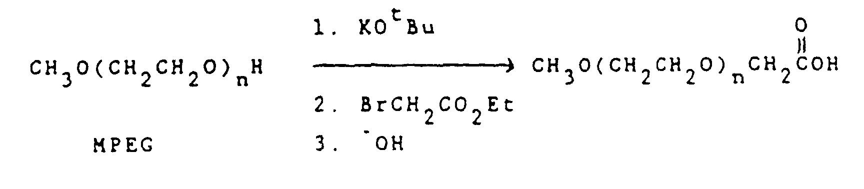

Figure 2 hereof schematically illustrates the synthesis of a PEG resin of Formula II. According to this method, an N^{α}-Fmoc, (N^{α}-9-fluorenylmethyloxycarbonyl), N^{δ}-Boc-Orn-PS (N^{δ}-tert-butyloxycarbonyl-ornithine- PS) resin (or a permutation on this theme) is deblocked selectively, and a monofunctional PEG-acid is coupled on to form a branch orthogonal to the ultimate direction of biopolymer chain growth. An ornithine residue (as shown in Figure 2) is used to link the PEG derivative to the solid support; however, other chemical moieties which have a carboxyl group and two amino groups can be used.

Polyethylene glycol-polystyrene (PEG-PS) graft supports made by the methods of this invention are particularly useful. PEG-PS supports made using the present PEG derivatives have several desirable characteristics for solid-phase applications: they swell in a variety of solvents, are stable under the condition used in most solid-phase synthesis, and behave well in both batch and column reactors used in solid-phase applications, in particular, solid-phase peptide synthesis.

Solid-phase peptide synthesis typically begins with covalent attachment of the carboxyl end of a first amino acid to the solid support. The carboxyl group of an N^{α}-protected amino acid is covalently linked to a handle moiety which is attached to the amino group of the PEG spacer arm at a point other than the PEG terminus (Figures 1 and 2). A "handle" is defined as a bifunctional spacer which serves to attach the initial amino acid residue to the polymeric support. One end of the handle incorporates a smoothly cleavable protecting group and the other end of the handle couples to the functionalized solid support. Handles which can be used with the present spacer arms in solid-phase peptide synthesis include, for example acid-labile p-alkoxybenzyl (PAB) handles, photolabile o-nitrobenzyl ester handles, and handles such as those described by Albericio et al., J. Org. Chem., 55:3730-3743 (1990) and references cited therein. The appropriate handles are coupled quantitatively in a single step onto the amino-functionalized supports to provide a general starting point of well-defined structures for peptide chain assembly. The handle protecting group is removed and the C-terminal residue of the N^{α}-protected first amino acid is coupled quantitatively to the handle. Once the handle is coupled to the solid-phase and the initial amino acid or peptide is attached to the handle, the general synthesis cycle proceeds. The synthesis cycle generally consists of deprotection of the N^{α}-amino group of the amino acid or peptide on the resin, washing, and, if necessary, a neutralization step, followed by reaction with a carboxyl-activated form of the next N^{α}-protected amino acid. The cycle is repeated to form the peptide or protein of interest. Solid-phase peptide synthesis methods using functionalized insoluble supports are well known. Merrifield, J. Am. Chem. Soc., 85:2149 (1963); Barany and Merrifield, In Peptides, Vol. 2, pp. 1-284 (1979); Barany et al., Int. J. Peptide Protein Res., 30:705-739 (1987).

The present PEG-derivatives are particularly useful as spacer arms, which separate the inert support from the reacting amino acids which are forming the peptide chain during the synthesis process. The choice of a spacer arm, which provides this distance, is a critical parameter in solid-phase applications.

In a preferred embodiment of the present invention, a variety of PEG spacer arms having an average molecular weight of about 2000 daltons were incorporated between amino functional groups on a polystyrene backbone and the point for attachment of the appropriate handles. The resultant PEG-PS graft supports contained approximately equal weight amounts PEG and PS. These supports shoved reproducible advantage over PS supports with regard to physical and chemical properties such as swelling and in synthesis of model peptides. The PEG-PS supports of this invention allow peptide synthesis to be carried out using acetonitrile as the solvent for all reaction steps and washes. The control experiments using PS and employing acetonitrile as the solvent resulted in no peptide products.

In another embodiment, comparative experiments were carried out in which the difficult acyl carrier protein 65-74 decapeptide sequence was synthesized using Fmoc-amino acids. The peptide was produced in higher purity on this new PEG-PS than on either PS, Tentagel™ (a trademark of Rapp Chem., Tubingen, Germany), or Pepsyn K™ (a trademark of Cambridge Research Biochem., Cambridge, England). The PEG-PS material proved highly suitable both for flow-through synthesis and batch operation. Negligible back-pressures were found even at high flow rates.

The general usefulness of the PEG-PS graft support was demonstrated by syntheses of a number of large, (e.g., having about 30 to 60 residues) complex peptide sequences, such as cecropin analogues, calcitonin, β-endorphin, corticotropin releasing factor, two zinc finger binding sequences, and several partial sequences of HIV-1 tat protein. The improvements in synthetic efficiency which resulted from use of the present PEG-PS linkers appear to originate from one or more of the following: (i) a spacer arm effect removing the reaction sites from the vicinity of the polymer backbone; (ii) a general environmental effect which modifies the hydrophobic nature of the resin with a concomitantly favorable influence on reaction rates; and (iii) a specific effect on conformationally difficult couplings due to decreased secondary structure (hydrogen bond formation).

The PEG derivatives can also be used in nucleic acid synthesis or sequencing as spacer arms or linkers between an inert support and the reacting nucleotide or nucleic acid. For example, the derivatives can be attached to polystyrene resins as described above and used in amidite-mediated DNA synthesis. The PEG-PS resin swells in acetonitrile, which is used as a solvent in the amidite coupling method.

The invention will now be further illustrated by the following examples:

### EXAMPLES

### Example 1. Preparation of a Carboxymethyl Derivative of Polyethylene Glycol Methyl Ether (MPEG)

A solution of polyethylene glycol methyl ether (MPEG), M_{W} 2000 (100 g, 50 mmol) in toluene (500 mL) was refluxed overnight for azeotropic drying, with water removed by a Dean-Stark trap attachment. The solution was cooled to 80°C, treated with potassium tert-butoxide (11.2 g, 0.1 mol), and then a mixture of ethyl bromoacetate (11 mL, 0.1 mol) and toluene (10 mL) was added over 30 minutes. Reaction continued for 8 hours at 90°C, following which the mixture was concentrated in a rotary evaporator to form a brown viscous melt. Dichloromethane (500 mL) was added to extract the polymer, and activated alumina (200 g) was added. After 30 minutes, the organic phase was collected upon filtration, partially concentrated (to - 100 mL), combined with ethyl ether (700 mL), and brought to -20°C for several hours. The precipitated polymer was collected by filtration, air-dried, and taken up in aqueous sodium hydroxide (1 N, 500 mL) for 3 hours, 25°C. This saponification reaction was quenched by acidification to pH 3 with concentrated aqueous HCl, and the product was extracted into dichloromethane (3 x 250 mL). The organic phase was dried (MgSO₄), partially concentrated (to - 100 mL), combined with ethyl ether (700 mL), and brought to -20°C for several hours. The final product was collected by filtration, and dried in vacuo over P₂O₅ for several days. Yield: 75 g (73%), titration of carboxyl groups 94% of theory. NMR data and elemental analysis in accord with structure.

### Example 2. Preparation of PEG-Orn(Boc)-PS Resin

An aminomethyl polystyrene resin support (6.0 g, 0.95 mmol/g, 5.7 mmol) was derivatized with N^{α}-Fmoc, N^{δ}-Boc-ornithine by standard procedures. The resin was swollen and washed with dichloromethane, neutralized with 5% DIEA in dichloromethane (1 x 5 min + 3 x 10 min), and washed with dichloromethane. Coupling of the Orn derivative was mediated by N,N'-diisopropylcarbodiimide (DIPCDI)/HOBt in DMF method (3 equiv. each, 15 min preactivation, 40 mL reaction volume), 3 hours. Washing followed with DMF and dichloromethane. A ninhydrin test on a sample was nearly negative, but unreacted sites were blocked by capping with acetic anhydride (4.5 mL, 5 equiv.) and triethylamine (7.0 mL, 5 equiv.) in DMF (20 mL), 30 minutes. The protected Orn-PS resin (0.78 mmol/g, matches theoretical loading as adjusted for expected weight gain) was washed with DMF, dichloromethane, and treated with piperidine-DMF (1:4, v/v) (2 + 10 min) to remove selectively the N^{α}-Fmoc group. Further washings with DMF, dichloromethane, 2-propanol, and DMF-dichloromethane (3:7, v/v) prepared the resin for coupling of the MPEG-acid derivative, produced as described in Example 1. A solution of the MPEG-acid (37 g, 18 mmol) in DMF-dichloromethane (3:7 v/v, 75 mL) was combined with HOBt (2.75 g, 18 mmol) and DIPCDI (2.83 mL, 18 mmol) for 15 minutes, and was then added to the H-Orn(Boc)-PS resin. Coupling proceeded for 5 hours, giving a slightly positive ninhydrin test. A second coupling with smaller amounts of MPEG-acid derivative and activating agents (9 mmol scale), and acetylation by already-described procedures followed. The final MPEG-Orn(Boc)-PS resin was washed with dichloromethane, DMF, dichloromethane and methanol, and dried in vacuo over P₂O₅ for 48 hours. There was obtained 16.9 g of resin (excellent agreement with theoretical weight gain) with a loading of 0.31 mmol/g, comprising PEG:PS:Orn - 0.57:0.36:0.07 by weight (based on elemental analysis and amino acid analysis). Similar results were obtained when BOP/HOBt/NMM in DMF protocols were used for activation and coupling.

### Example 3. Continuous Flow Peptide Synthesis with Polyethylene Glycol-Polystyrene Graft (PEG-PS)

PEG-Orn(Boc)-PS prepared as described in Example 2 was treated with TFA-dichloromethane (1:1) (5 + 25 min) to remove selectively the N^{δ}-Boc group. There followed washing with dichloromethane, neutralization with 5% DIEA in dichloromethane and dichloromethane. An Fmoc-PAL handle was added, and a MilliGen/Biosearch model 9050 peptide synthesizer was used with a DIPCDI + HOBt coupling protocol (0.05 M concentrations), to make the challenging deca-alanyl-valine sequence. In a consecutive run, a normal polystyrene support was used to make the same sequence. The purity of the peptide based on HPLC was 77% when PEG-PS was used, as compared to 53% when PS was used.

## Claims

1. A method of producing a resin for solid-phase peptide synthesis, comprising the steps of:
a) providing an amino-functionalized solid support linked to a protected diamino monocarboxylic acid;
b) removing a protecting group from one of the two N^{ω}-amino moieties; and
c) coupling a monofunctional carboxyl terminal polyethylene glycol derivative to the deprotected N^{ω}-amino group to thereby produce a resin;
wherein the resin is represented by the formula 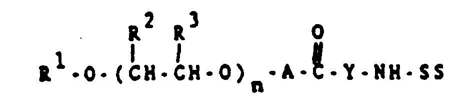 wherein Y is a diamino monocarboxylic acid residue that can optionally be protected by a N^{ω} protecting group; n is an integer from 5 to 150; SS is a solid support; A is a straight chain or branched C1-C10 alkyl group; and R¹, R² and R³ are independently selected from the group consisting of hydrogen, alkyl groups and aryl groups.

2. The method of claim 1, wherein the solid support is selected from amino-functional membranes, porous glass, silica, polystyrenes, polydimethylacrylamides, cotton and paper.

3. The method of claim 2, wherein the polystyrene is selected from the group consisting of amino-polystyrene, aminomethyl polystyrene, aminoacyl polystyrene, and p-methylbenzhydrylamine polystyrene.

4. The method of claim 1, wherein R¹, R² and R³ are independently H or R¹ is a methyl group.

5. The method of claims 1, wherein A is independently selected from the group consisting of ethylene, propylene, isopropylene, butylene and isobutylene.

6. The method of claim 1-5, wherein the N^{ω}-protected diamino monocarboxylic acid is ornithine.

7. The method of claims 1-6, wherein the monofunctional carboxyl terminal polyethylene glycol derivative is formed by reacting an amino-derivatized polyethylene glycol with a dicarboxylic acid or dicarboxylic acid anhydride.

8. The method of claim 7, wherein the dicarboxylic acid is selected from the group consisting of maleic acid, succinic acid, glutaric acid, and adipic acid.

9. The method of claim 7, wherein the dicarboxylic acid is selected from the group consisting of maleic acid anhydride, succinic acid anhydride, glutaric acid anhydride, and phthalic acid anhydride.

10. The method of claims 1-6, wherein the monofunctional carboxyl terminal polyethylene glycol derivative is formed by the steps of
d) reacting monomethyl-polyethylene glycol with ethyl bromoacetate, 4-bromovalerate or isocyanate; and
e) subjecting the product of step d) to saponification.

## Patentansprüche

1. Ein Verfahren zur Herstellung eines Harzes zur Festphasen Peptid-Synthese, umfassend die Schritte:
a) Bereitstellen eines amino-funktionalisierten, festen Trägers, der mit einer geschützten Diamino-Monocarbonsäure verbunden ist;
b) Entfernen einer Schutzgruppe von einer der zwei N^{ω}-Amino-Komponeneten; und
c) Koppeln eines monofunktionellen, carboxylterminalen Polyethylenglykolderivates an die entschützte N^{ω}-Aminogruppe um dadurch ein Harz herzustellen;
wobei das Harzes dargestellt ist durch die Formel 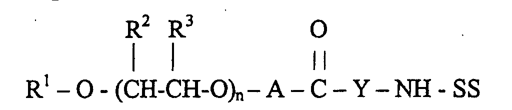 worin Y ein Diamino-Monocarbonsäurerest ist, der ggf. durch eine N^{ω}-Schutzgruppe geschützt sein kann; n eine ganze Zahl von 5 bis 150 ist; SS ein fester Träger ist; A eine linerare Kette oder verzweigte C1-C10 Alkylgruppe ist; und R¹, R² und R³ unabhängig voneinander ausgewählt sind aus der Gruppe bestehend aus Wasserstoff, Alkylgruppen und Arylgruppen.

2. Das Verfahren nach Anspruch 1, worin der feste Träger ausgewählt ist aus amino-funktionellen Membranen, porösem Glas, Silikaten, Polystyrolen, Polydimethylacrylamiden, Baumwolle und Papier.

3. Das Verfahren nach Anspruch 2, worin das Polystyrol ausgewählt ist aus der Gruppe bestehend aus Amino-Polystyrol, Aminomethyl-Polystyrol, Aminoacyl-Polystyrol und p-Methylbenzhydrylamin-Polystyrol.

4. Das Verfahren nach Anspruch 1, worin R¹, R² und R³ unabhängig voneinander Wasserstoff sind oder R¹ eine Methylgruppe ist.

5. Das Verfahren nach Anspruch 1, worin A ist unabhängig ausgewählt aus der Gruppe bestehend aus Ethylen, Propylen, Isopropylen, Butylen und Isobutylen.

6. Das Verfahren nach Ansprüchen 1-5, worin die N^{ω}-geschützte Diaminomonocarbonsäure Ornithin ist.

7. Das Verfahren nach Ansprüchen 1-6, worin das monofunktionelle, carboxylterminale Polyethylenglykolderivat gebildet wird durch Reaktion eines aminoderivartisierten Polyethylenglykols mit einer Dicarbonsäure oder einem Dicarbonsäureanhydrid.

8. Das Verfahren nach Anspruch 7, worin die Dicarbonsäure ist ausgewählt aus der Gruppe bestehend aus Maleinsäure, Bernsteinsäure, Glutarsäure und Adipinsäure.

9. Das Verfahren nach Anspruch 7, worin die Dicarbonsäure ist ausgewählt aus der Gruppe bestehend aus Maleinsäureanhydrid, Bernsteinsäureanhydrid, Glutarsäureanhydrid und Phthalsäureanhydrid.

10. Das Verfahren nach Ansprüchen 1-6, worin das monofunktionelle, carboxylterminale Polyethylenglykolderivat gebildet wird durch die Schritte
d) Reagieren von Monomethyl-polyethylenglykol mit Ethylbromacetat, 4-Bromvalerat oder Isocyanat; und
e) das Produkts von Schritt d) der Verseifung unterworfen wird.

## Revendications

1. Procédé de préparation d'une résine pour la synthèse de peptides en phase solide comprenant les étapes consistant à :
a) préparer un support solide amino-fonctionnalisé lié à un acide diaminomonocarboxylique protégé ;
b) supprimer un groupe protecteur de l'une des deux fractions N^{ω}-amino ; et
c) coupler un dérivé polyéthylène glycol monofonctionnel à extrémité carboxyle au groupe N^{ω} -amino déprotégé de manière à préparer une résine ;
dans lequel la résine est représentée par la formule 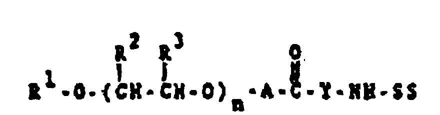 où Y est un résidu acide diaminomonocarboxylique pouvant être facultativement protégé par un groupe protecteur de N^{ω} ; n est un entier de 5 à 150 ; SS est un support solide ; A est un groupe alkyle en C₁-C₁₀ à chaîne linéaire ou ramifiée, et R₁, R₂ et R₃ sont indépendamment choisis parmi le groupe comprenant de l'hydrogène, les groupes alkyle et groupes aryle.

2. Procédé selon la revendication 1, dans lequel le support solide est choisi parmi des membranes amino-fonctionnelles, le verre poreux, la silice, les polystyrènes, les polyméthylacrylamides, le coton et le papier.

3. Procédé selon la revendication 2, dans lequel le polystyrène est choisi parmi le groupe comprenant l'amino-polystyrène, l'aminométhyle le polystyrène, l'aminoacyle polystyrène, et le p-méthylbenzhydrylamine polystyrène.

4. Procédé selon la revendication 1, dans lequel R₁ R₂ et R₃ sont indépendamment H ou R₁ est un groupe méthyle.

5. Procédé selon la revendication 1, dans lequel A est indépendamment choisi parmi le groupe comprenant l'éthylène, le propylène, l'isopropylène, le butylène et l'isobutylène.

6. Procédé selon les revendications 1 à 5, dans lequel l'acide diaminomonocarboxylique N^{ω}-protégé est l' ornithine.

7. Procédé selon les revendications 1 à 6, dans lequel le dérivé monofonctionnel à extrémité carboxyle est formé en faisant réagir un polyéthylène glycol dérivé d'amine avec un acide dicarboxylique ou avec un anhydride d'acide dicarboxylique.

8. Procédé selon la revendication 7, dans lequel l'acide dicarboxylique est choisi parmi le groupe comprenant l'acide maléique, l'acide succinique, l'acide glutarique et l'acide adipique.

9. Procédé selon la revendication 7, dans lequel l'acide dicarboxylique est choisi parmi l'anhydride d'acide maléique, l'anhydride d'acide succinique, l'anhydride d'acide glutarique, et l'anhydride d'acide phthalique.

10. Procédé selon les revendications 1 à 6, dans lequel le dérivé polyéthylène glycol monofonctionnel à extrémité carboxyle est formé par les étapes consistant à
d) faire réagir du monométhylpolyéthylène glycol avec du bromoacétate, du 4-bromovalérate ou de l'isocyanate d'éthyle ; et
e) soumettre le produit de l'étape d) à une saponification.
